# EUROPEAN PATENT SPECIFICATION

(11) **EP 1 080 381 B1**
(45) Date of publication and mention of the grant of the patent: **27.07.2005**
(21) Application number: 99915252.3
(22) Date of filing: 01.04.1999
(51) Int. Cl.: G02B 1/04, C08F 20/30, C08F 20/36, A61L 27/00

(54) **HIGH REFRACTIVE INDEX OPHTHALMIC DEVICE MATERIALS PREPARED USING A POST-POLYMERIZATION CROSS-LINKING METHOD**
IN EINEM VERFAHREN MIT VERNETZUNG DURCH NACHPOLMERISATION HERGESTELLTES MATERIAL FÜR OPHTHALMOLOGISCHE GERÄTE MIT HOHEM BRECHUNGSINDEX
MATERIAUX POUR DISPOSITIFS OPHTALMIQUES A INDICE DE REFRACTION ELEVE PREPARES A L'AIDE D'UN PROCEDE DE RETICULATION EFFECTUE APRES LA POLYMERISATION

(30) Priority: 15.04.1998 US 81874 P
(43) Date of publication of application: 07.03.2001
(73) Proprietor: Alcon Manufacturing Ltd., Fort Worth, Texas 76134-2099 (US)
(72) Inventor: LEBOEUF, Albert, R., Fort Worth, TX 76133 (US)
(74) Representative: Best, Michael, Dr.
(86) International application number: PCT/US1999/007362
(87) International publication number: WO 1999/053348

(56) References cited:
- EP-A- 0 308 130
- EP-A- 0 345 994
- EP-A- 0 514 096
- WO-A-94/11764
- FR-A- 2 765 583

## Description

### FIELD OF THE INVENTION

This invention relates to a method of preparing high refractive index ophthalmic device materials. In particular, the present invention relates to a two-stage method in which ophthalmic device materials are first polymerized and then cross-linked.

### BACKGROUND OF THE INVENTION

The two most common types of polymerization initiators for ophthalmic device materials are thermal initiators and photoinitiators. Typical thermal initiators, including free radical initiators such as peroxides, initiate polymerization as temperature is increased. In some cases, two or three temperature tiers are involved such that curing involves a schedule of temperature/time combinations. Thermal initiation generally requires holding the monomer composition at elevated temperatures for lengthy periods of time. Total cure times of twenty-four hours are not unusual. See, for example, U.S. Patent No. 5,290,892.

Photoinitiators generally offer the advantage of relatively short cure times and, unlike thermal initiators, can be used at ambient conditions, eliminating the need for high-temperature equipment or special ovens. Photoinitiators are activated by radiation of one or more specified wavelengths, rather than heat. Photoinitiation of ophthalmic lens materials is known. See, for example, U.S. Patent No. 5,290,892.

The most common types of photoinitiators known or used for curing ophthalmic lens polymers are probably UV-sensitive photoinitiators. UV-sensitive photoinitiators are, however, generally not suitable for use with lens materials that contain a UV-absorbing chromophore. UV-absorbing chromophores present in an ophthalmic lens composition can interfere with the ability of UV-sensitive photoinitiators to efficiently cure the composition. Today, UV-absorbing chromophores are frequently incorporated in ophthalmic lens materials in order to reduce or block UV light from reaching the retina. Although methods are known for temporarily "blocking" UV absorbing chromophores during processing, thereby preventing interference with a UV-initiator, these methods require that the UV-absorber be "un-blocked" after the composition is cured. The UV chromophore can be "un-blocked" by either chemical or thermal means. "Un-blocking" is generally complicated and can add 4 - 6 hours to processing times, offsetting some or all of the time advantages offered by photoinitiators.

In addition to UV-sensitive photoinitiators, visible-light initiators are also known. For example, U.S. Patent No. 5,224,957 discloses photopolymerizable compositions useful in forming an intraocular lens in situ. The compositions are delivered into the natural lens capsule or a thin plastic shell substitute and then polymerized. The reference compositions contain 90 - 99.99% by weight of at least one polyfunctional acrylic and/or methacrylic acid ester. Suitable acid esters include bisphenol A or bishydroxypolyalkoxy bisphenol A derivatives lengthened with ethylene oxide or propylene oxide. The compositions of the '957 patent are cured using photoinitiators which absorb light in the range 400 - 500 nm. Suitable initiators include alpha-diketones, in particular camphorquinone, benzil and phenanthrene quinone, and mono and bisacylphosphine oxides.

International Patent Application Publication No. WO 96/28762 also discloses photocurable compositions comprising acrylic components. The compositions contain specified amounts of di(meth)acrylates, poly(meth)acrylates, urethane(meth)acrylates, and oligomeric di(meth)acrylates based on bisphenol A or bisphenol F. The photoinitiator may be "any photoinitiator which forms free radicals when irradiated suitably." Suitable classes include benzoin ethers; acetophenones; benzil; anthraquinones; benzoylphosphine oxides (e.g., 2,4,6-trimethylbenzoyldiphenylphosphine oxide); benzophenones. Photoinitiators particularly suitable for use with argon ion lasers include 2,4,6- trimethylbenzoyldiphenylphosphine oxide.

Some ophthalmic devices are obtained by a monomer cast polymerization method. In such a method, the monomer solution is cast directly into a mold of desired shape and then polymerized or cured, followed by any machining or polishing, etc. See, for example, U.S. Patent Nos. 4,921,205 and 5,290,892.

In other cases, ophthalmic device materials are formed by first preparing a "prepolymer" or partially cured material, followed by further curing. See, for example, U.S. Patent No. 5,374,663 describing a prepolymer process for producing a U.V. absorber-containing intraocular lens material in which a monomer solution comprising a lens-forming monomer, an U.V. absorber and a polymerization initiator is introduced into a reactor and heated for a length of time and at a temperature sufficient to obtain a prepolymer of high viscosity. Thereafter, the prepolymer is filtered, cast into a cell or mold and further heated for a time at a temperature sufficient to obtain a transparent lens material.

According to the '663 patent, the prepolymer process has the advantage that the prepolymer scarcely leaks out of the cell or mold because of its high viscosity, and that the degree of shrinkage in the step of obtaining a lens material from the prepolymer is small. On the other hand, the prepolymer process has some problems as well, including (i) the control of the polymerization degree and viscosity of the prepolymer obtained in the first polymerization step, and (ii) when a cross-linking monomer is contained in the material, an insoluble polymer is formed in the prepolymer step, making any filtration treatment difficult or impossible, and the polymer produced after the further curing step becomes "non-uniform."

WO 94/11764 discloses a method for polymerizing a hydrophobic acrylic monomer. The starting material contains an ingredient having more than one unsaturated bond such as ethylene glycol dimethacrylate.

WO 97/09170 discloses a two stage method for producing ophthalmic lenses. The method involves a conventional cross linking agent.

### SUMMARY OF THE INVENTION

The present invention relates to a method for preparing acrylic, high refractive index ophthalmic device materials. The ophthalmic device materials comprise at least one aryl acrylic hydrophobic monomer, a first stage polymerization initiator selected from the group consisting of low-temperature thermal initiators and photoinitiators, and a second stage cross-linking agent selected from the group consisting of dibenzoyl peroxide; substituted dibenzoyl peroxide compounds, and dicumyl peroxide. The monomers used to form the ophthalmic device materials do not contain any ingredient having more than one unsaturated site, as such ingredients will cause premature cross-linking.

According to the present invention, the ophthalmic device material is prepared using a two-stage process. In the first stage, the material is polymerized such that the second stage cross-linking agent is not activated. In the second stage, the material is cross-linked by activating the cross-linking agent. The two-stage process of the present invention can provide enhanced control of material shrinkage and stress problems associated with cast molding operations compared to single stage curing processes.

### DETAILED DESCRIPTION OF THE INVENTION

As used herein, "high refractive index" means a refractive index of about 1.50 or greater when measured at room temperature using an Abbe' refractometer at 589 nm (Na light source).

As used herein, "low-temperature thermal initiator" means a thermal initiator that has an activation temperature lower than the activation temperature of the chosen second stage cross-linking agent.

According to the present invention, acrylic, high refractive index ophthalmic device materials are prepared in two stages. In the first stage, the device material is polymerized. In the second stage, the device material is cross-linked.

The ophthalmic device materials of the present invention comprise at least one compound of Formula I below. 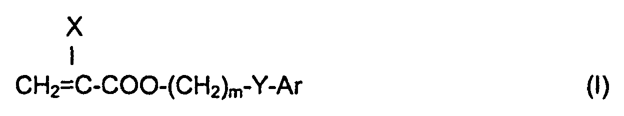 wherein:
X is H or CH₃;
m is 0-10;
Y is nothing, O, S, or NR wherein R is H, CH₃, CₙH₂ₙ₊₁ (n=1-10) iso OC₃H₇, C₆H₅, or CH₂C₆H₅;
Ar is any aromatic ring which can be unsubstituted or substituted with CH₃, C₂H₅, n-C₃H₇, iso-C₃H₇, OCH₃, C₆H₁₁, Cl, Br, C₆H₅, or CH₂C₆H₅.

Monomers of Formula I are known and include, but are not limited to: 2-phenoxyethyl acrylate; 2-phenylethylthio acrylate; 2-phenylethylamino acrylate; phenyl acrylate; benzyl acrylate; 2-phenylethyl acrylate; 3-phenylpropyl acrylate; 3-phenoxypropyl acrylate; 4-phenylbutyl acrylate; 4-phenoxybutyl acrylate; 4-methylphenyl acrylate; 4-methylbenzyl acrylate; 2-2-methylphenylethyl acrylate; 2-3-methylphenylethyl acrylate; 2-4-methylphenylethyl acrylate; and their corresponding methacrylate compounds. These acrylic/methacrylic monomers and others are disclosed in U.S. Patent No. 5,290,892, the entire contents of which are hereby incorporated by reference.

Preferred monomers of Formula (I) are those where m is 2-4; Y is nothing or O; and Ar is phenyl. Most preferred are 2-phenylethyl acrylate, 2-phenoxyethyl acrylate, 3-phenylpropyl acrylate, 3-phenoxypropyl acrylate, 4-phenylbutyl acrylate, and 4-phenoxybutyl acrylate, and their corresponding methacrylate compounds.

The ophthalmic device materials of the present invention preferably contain at least two monomers of Formula I, wherein at least one is a methacrylate monomer (X = CH₃) and at least one is an acrylate monomer (X = H). The exact amount of monomer of Formula I present in the acrylic, high refractive index ophthalmic device materials of the present invention will vary depending upon the identity of the monomer(s) of Formula I, the identity of any other device-forming monomer(s) present in the materials, and the desired mechanical properties. For example, foldable intraocular lenses are preferably made from polymers having a glass transition temperature no greater than normal room temperature, e.g., about 20 - 25 °C, in order that the lenses can be rolled or folded conveniently at room temperature. Materials having a glass transition temperature of about 15 °C or less are even more preferred for foldable intraocular lens applications. Glass transition temperature is determined at room temperature using a differential scanning calorimeter at a heating rate of 10°C/min.

Additionally, materials exhibiting an elongation of at least 150% when measured at room temperature using an Instron tensile tester at a cross-head speed of 50 cm/min) are preferred for use in foldable intraocular lenses because such lenses must exhibit sufficient strength to allow them to be folded without fracturing. For foldable intraocular lens applications, polymers having an elongation of at least 200% are more preferred.

In general, the acrylic, high refractive index ophthalmic device materials of the present invention preferably contain at least 50% (w/w) of monomer(s) of Formula I. In a more preferred embodiment, the device materials will contain one or more monomers of Formula I in an amount totaling 70% (w/w) or more, and most preferably, 80% (w/w) or more.

Device-forming monomers other than those of Formula I optionally may be included in the materials of the present invention. Many such ophthalmic device-forming monomers are known. Any known device-forming monomer may be used if it is compatible with the monomer(s) of Formula I present in the ophthalmic device material and does not prevent the ability of the stage 1 polymerization initiator to cure the material such that the material contains no cross-linking or is substantially free of cross-linking. Suitable device-forming monomers other than those of Formula I include, but are not limited to: C₁ - C₈ alkylacrylates, C₁ - C₈ cycloalkylacrylates, N-alkylacrylamides (where alkyl = C₁ - C₄), phenoxyalkylacrylates (where alkyl = C₁ - C₈), and their corresponding methacrylates. Suitable device-forming monomers other than those of Formula I also include N-vinylpyrrolidone. See U.S. Patent No. 5,331,073, the entire contents of which are hereby incorporated by reference, for examples of device-forming materials other than those of Formula 1.

As in the case of the monomer(s) of Formula I, the amount of any other device-forming monomers present in the ophthalmic device materials of the invention will vary depending upon the identity of the monomer(s) of Formula I, the identity of the optional device-forming monomer(s), and the mechanical properties desired for the finished ophthalmic material. In general, for foldable intraocular lens applications, the ophthalmic device materials of the present invention preferably contain about 45% (w/w) or less, and more preferably about 30% (w/w) or less, of device-forming monomers other than those of Formula I.

The ophthalmic device materials also comprise a first stage polymerization initiator selected from the group consisting of low temperature thermal initiators and photoinitiators. Suitable low temperature thermal initiators include azo free-radical initiators, such as 2,2'-azobis(isobutyronitrile) ["AIBN"] and 2,2'-azobis(2,4-dimethylvalerontrile). Suitable photoinitiators include UV-and blue-light photoinitiators. Many such photoinitiators are known. Preferred blue-light photoinitiators are benzoylphosphine oxide initiators, such as 2,4,6-trimethyl-benzoyldiphenylophosphine oxide; bis-(2,6-dichlorobenzoyl)-4-N-propylphenyl-phosphine oxide; and bis-(2,6-dichlorobenzoyl)-4-N-butylphenylphosphine oxide. Most preferred is 2,4,6-trimethylbenzoyldiphenylophosphine oxide, commercially available as Lucirin® TPO from BASF Corporation (Charlotte, North Carolina). See, for example, commonly-assigned, co-pending U.S. Patent Application Serial No. 08/908,229, filed on August 7, 1997, the entire contents of which are hereby incorporated by reference.

The amount of the first stage polymerization initiator in the device materials of the present invention will depend upon the identity of the other ingredients in the materials, the curing conditions, etc. In general, however, the amount of first stage polymerization initiator contained in the mixture to be polymerized in stage of the present invention will be about 3 % (w/w) or less, preferably about 2 % (w/w) or less, and most preferably about 1 % (w/w).

In addition to the device-forming monomer(s) (i.e., monomers of Formula I and any other device forming monomers) and the first stage polymerization initiator, the ophthalmic device materials of the present invention contain a second stage cross-linking agent. The second stage cross-linking agent is selected from the group consisting of dibenzoyl peroxide, substituted dibenzoyl peroxide compounds, and dicumyl peroxide, many of which are commercially available. For example, dicumyl peroxide is available from Hercules Incorporated (Wilmington, Delaware). Suitable substituted dibenzoyl peroxide compounds include 2,4-dichlorodibenzoylperoxide. Dicumyl peroxide is preferred for use with ophthalmic device materials comprising 2-phenylethyl acrylate and 2-phenylethyl methacrylate.

The amount of the second stage cross-linking agent contained in the device materials of the present invention will depend upon, among other factors, the degree of cross-linking desired. In general, however, the amount of second stage cross-linking agent in the ophthalmic device materials will be about 2 - 5 % (w/w), and preferably about 2.5 - 4 % (w/w).

In order to prevent premature cross-linking, the ophthalmic device materials of the present invention do not contain any ingredient having more than one unsaturated bond. Such ingredients include the common cross-linking monomers ethyleneglycol dimethacrylate; diethylene glycol dimethacrylate; ethyleneglycol diacrylate; allyl methacrylates; allyl acrylates; 1,3-propanediol dimethacrylate; 1,6-hexanediol dimethacrylate; 1,4-butanediol dimethacrylate; polyethyleneoxide mono- and diacrylates; and the like.

Ultraviolet absorbing chromophores are optionally included in the ophthalmic device materials of the present invention. Such chromophores prevent or inhibit UV light from damaging the eye. The ultraviolet absorbing chromophore in the device material of the present invention can be any compound which absorbs light having a wavelength shorter than about 400 nm, but does not absorb any substantial amount of visible light, and which is compatible with the device-forming monomer(s) present in the material . The ultraviolet absorbing compound is incorporated into the monomer mixture and is entrapped in the polymer matrix when the monomer mixture is polymerized. Suitable ultraviolet absorbing compounds include substituted benzophenones, such as 2-hydroxybenzophenone, and 2-(2-hydroxyphenyl)-benzotriazoles. It is preferred to use an ultraviolet absorbing compound that is copolymerizable with the device-forming monomers described above so that it will be covalently bound to the polymer matrix. In this way, possible leaching of the ultraviolet absorbing compound out of the device and into the interior of the eye is minimized. Suitable copolymerizable ultraviolet absorbing compounds are the substituted 2-hydroxybenzophenones disclosed in U.S. Patent No. 4,304,895 and the 2-hydroxy-5-acryloxyphenyl-2H-benzotriazoles disclosed in U.S. Patent No. 4,528,311. The most preferred ultraviolet absorbing compound is 2-(3'-methallyl-2'-hydroxy-5'-methyl phenyl) benzotriazole.

If the ophthalmic device material does include a UV-absorber, it is unlikely that a UV polymerization initiator may be used as the first stage polymerization initiator. In such cases, the first stage polymerization initiator will likely have to be either a low temperature thermal initiator or a blue-light initiator in order to avoid interference with the UV-absorber.

Blue-light absorbing compounds are also optionally included in the device materials of the present invention. If a blue-light absorbing compound, e.g. a yellow dye, is included in the device material of the present invention, then the first stage polymerization initiator will likely not be a blue-light photoinitiator. In the event the device material contains both a UV-absorber and a blue-light absorbing compound, the first stage polymerization initiator will likely be a low temperature thermal initiatior. Preferably, blue-light absorbers are copolymerizable with the device-forming monomers. Suitable polymerizable blue-light blocking chromophores include those disclosed in U.S. Patent No. 5,470,932.

The device materials of this invention are prepared by forming a mixture comprising the device-forming monomer(s) (monomer(s) of Formula I and any optional device-forming monomer(s)), the first stage polymerization initiator and the second stage cross-linking agent, along with any UV- or blue-light absorbing compounds and any other suitable ingredients, in the desired proportions. The mixture can then be introduced into a mold of desired shape to form an ophthalmic device. Alternatively, the mixture can be cast in sheets from which the finished form can be obtained by compression molding (generally with mild pre-heating).

In either case (direct cast molding in final form or casting in sheets for subsequent molding), the ophthalmic device material is polymerized in the first stage of the present invention by activating the first stage polymerization initiator (e.g., using heat, UV- or blue-light). In the case where the first stage polymerization initiator is a low temperature thermal initiator and the second stage cross-linking agent is dicumyl peroxide, the thermal initiator may be activated by exposure to temperatures of up to approximately 50 °C or so without activating the dicumyl peroxide. In the case where the second stage cross-linking agent is a dibenzoyl peroxide or a substituted dibenzoyl peroxide, the curing temperature for stage 1 will generally be about 40 °C or less. The curing parameters, e.g., length of exposure and temperature or intensity of light source, are preferably chosen to accomplish complete polymerization.

After the ophthalmic device material is polymerized in stage 1, it is cross-linked in stage 2 of the present invention. Cross-linking is achieved by activating the second stage cross-linking agent using heat. The temperature and length of exposure to heat are determined by the identity and amount of the second stage cross-linking agent and the desired degree of cross-linking to be achieved (i.e., the desired physical properties of the ophthalmic device materials). In general, however, the temperature will be about 90 °C or more where the second stage cross-linking agent is a dibenzoyl peroxide or a substituted dibenzoyl peroxide. In the case where the second stage cross-linking agent is dicumyl peroxide, the activation temperature will be about 125 °C or greater. The duration of heating to achieve the second-stage cross-linking is preferably about four times the half-life of the second stage cross-linking agent at the chosen activation temperature. In the case of dicumyl peroxide and an activation temperature of approximately 135 °C, the duration of heating is approximately 4 hours.

The ophthalmic device materials prepared according to the present invention may be used to make almost any type of ophthalmic lens, including contact lenses, intracomeal lenses and intraocular lenses. Ophthalmic lenses constructed of the disclosed materials can be of any design, but are preferably intraocular lenses (IOLs) capable of being rolled or folded and inserted through a relatively small incision. For example, the IOLs can be of what is known as a one piece or multipiece design. Typically, an IOL comprises an optic and at least one haptic. The optic is that portion which serves as the lens and the haptics are attached to the optic and are like arms that hold the optic in its proper place in the eye. The optic and haptic(s) can be of the same or different material. Haptics may be attached to the optic using conventional techniques. In a single piece lens, the optic and the haptics are formed out of one piece of material. Depending on the material, the haptics are then cut, or lathed, out of the material to produce the IOL. In addition to ophthalmic lenses, the materials prepared according to the methods of the present invention may also be used to make other ophthalmic devices including, but not limited to, keratoprostheses and corneal inlays or rings.

Molding and drilling operations are easily carried out if the device, e.g., an IOL optic, is molded between two polypropylene mold halves. The mold containing the cured device material is then placed on a lathe and cut to the desired shape. The mold may then be easily mounted to carry out any drilling operations prior to removing the mold halves. Both the lathing and drilling operations may be facilitated by cooling the mold/device in a freezer to less than 10 °C and preferably less than 0 °C prior to each of these operations. If premature release of one or both mold halves occurs, it may be necessary to use clamps or alternative mold materials or to pretreat the surface of the mold halves.

The invention will be further illustrated by the following examples which are intended to be illustrative, but not limiting.

### EXAMPLES

The ophthalmic device materials shown below in Table 1 were prepared as follows:
Example 1 was prepared by heating at 70 °C for 7 hours, followed by heating at 100°C for 7 hours.
Example 2 was prepared by heating at 135 °C for 17.5 hours.
Examples 3 - 14 were prepared using the two-stage method of the present invention. In stage 1, the ingredients were mixed, transferred to 1-mm thick, slab, polypropylene mold, and polymerized by exposure to blue light (Palatray CU/14 mW/cm²) for 15 minutes. In stage 2, the materials remained in the same slab molds and were cross-linked by heating at 135 C for 4, 6 or 10 hours as indicated.

In all cases (Examples 1 - 14) the ophthalmic device materials were vacuum degassed for approximately 10 minutes immediately prior to filling and sealing the polypropylene slab molds.

The amount of each of the ingredients is expressed in % w/w.

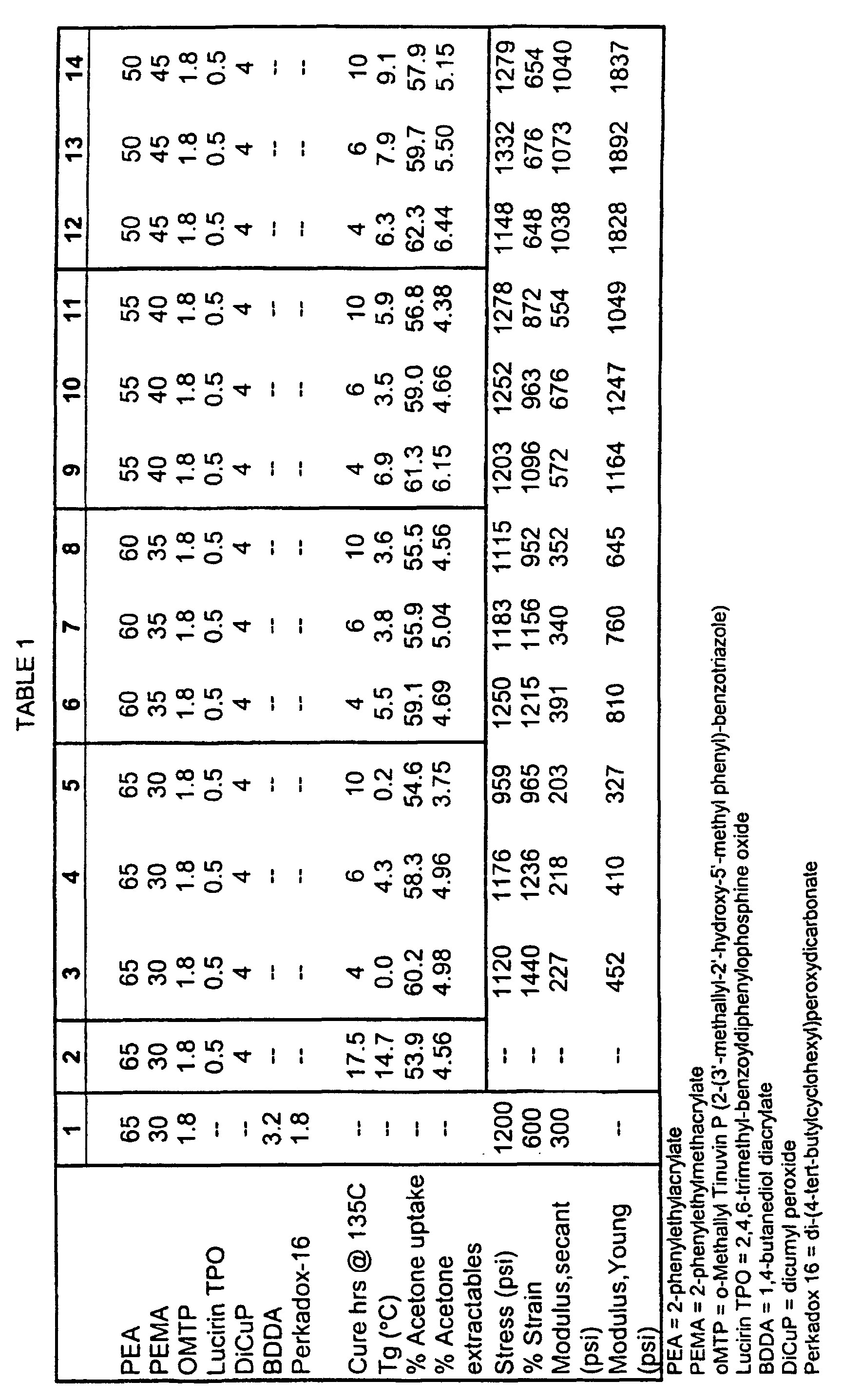

The invention has been described by reference to certain preferred embodiments; however, it should be understood that it may be embodied in other specific forms or variations thereof without departing from its spirit or essential characteristics. The embodiments described above are therefore considered to be illustrative in all respects and not restrictive, the scope of the invention being indicated by the appended claims rather than by the foregoing description.

## Claims

1. A two-stage method for preparing an acrylic, high refractive index ophthalmic device material, wherein the ophthalmic device material comprises
(i) at least one aryl acrylic hydrophobic monomer of the formula 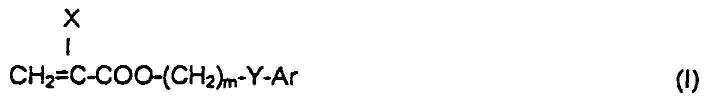 wherein:
X is H or CH₃;
m is 0-10;
Y is nothing, O, S, or NR wherein R is H, CH₃, CₙH₂ₙ₊₁ (n=1-10) iso OC₃H₇, C₆H₅, or CH₂C₆H₅;
Ar is any aromatic ring which can be unsubstituted or substituted with CH₃, C₂H₅, n-C₃H₇, iso-C₃H₇, OCH₃, C₆H₁₁, Cl, Br, C₆H₅, or CH₂C₆H₅;
(ii) a first stage polymerization initiator selected from the group consisting of low temperature thermal polymerization initiators and photoinitiators; and
(iii) a second-stage cross-linking agent selected from the group consisting of dibenzoyl peroxide; substituted dibenzoyl peroxide compounds, and dicumyl peroxide; and
wherein the first stage of the method comprises polymerizing the ophthalmic device material by activating the first stage polymerization initiator without activating the second stage cross-linking agent; and the second stage of the method comprises cross-linking the ophthalmic device material by activating the second stage cross-linking agent using heat, provided that the ophthalmic device material does not contain any ingredient having more than one unsaturated bond.

2. The method of Claim 1 wherein the aryl acrylic hydrophobic monomers is selected from the group consisting of 2-phenoxyethyl acrylate; 2-phenylethylthio acrylate; 2-phenylethylamino acrylate; phenyl acrylate; benzyl acrylate; 2-phenylethyl acrylate; 3-phenylpropyl acrylate; 3-phenoxypropyl acrylate; 4-phenylbutyl acrylate; 4-phenoxybutyl acrylate; 4-methylphenyl acrylate; 4-methylbenzyl acrylate; 2-2-methylphenylethyl acrylate; 2-3-methylphenylethyl acrylate; 2-4-methylphenylethyl acrylate; and their corresponding methacrylate

3. The method of Claim 1 wherein m is 2-4; Y is nothing or O; and Ar is phenyl.

4. The method of Claim 1 wherein the ophthalmic device material comprises at least two aryl acrylic hydrophobic monomers of Formula (I) and further wherein at least one of the aryl acrylic hydrophobic monomers is a methacrylate monomer and at least one of the aryl acrylic hydrophobic monomers is an acrylate monomer.

5. The method of Claim 4 wherein the ophthalmic device materials comprise a total of at least 50 % (w/w) of aryl acrylic hydrophobic monomers of Formula (I).

6. The method of Claim 5 wherein the ophthalmic device materials comprise a total of at least 70 % (w/w) of aryl acrylic hydrophobic monomers of Formula (I).

7. The method of Claim 1 wherein the ophthalmic device material further comprises one or more monomers selected from the group consisting of C₁ - C₈ alkylacrylates; C₁ - C₈ cycloalkylacrylates; C₁ - C₈ N-alkylacrylamides; C₁ - C₈ phenoxyalkylacrylates; and their corresponding methacrylates.

8. The method of Claim 1 wherein the first stage polymerization initiator is selected from group consisting of 2,2'-azobis(isobutyronitrile); 2,2'-azobis(2,4-dimethylvalerontrile); UV-photoinitiators; and blue-light photoinitiators.

9. The method of Claim 1 wherein the first stage polymerization initiator is a benzoylphosphine oxide initiators.

10. The method of Claim 1 wherein the amount of the first stage polymerization initiator is about 3 % (w/w) or less.

11. The method of Claim 1 wherein the second stage cross-linking agent is selected from the group consisting of dibenzoyl peroxide; 2,4-dichlorodibenzoylperoxide; and dicumyl peroxide.

12. The method of Claim 11 wherein the second stage cross-linking agent is dicumyl peroxide.

13. The method of Claim 1 wherein the amount of the second stage cross-linking agent is about 2 - 5 % (w/w).

14. The method of Claim 1 wherein the ophthalmic device material further comprises one or more ingredients selected from the group consisting of UV absorbing compounds and blue-light absorbing compounds.

15. An ophthalmic device material obtainable by the method of Claim 1.

## Patentansprüche

1. Zweistufenverfahren zur Herstellung eines Acrylmaterials für ophthalmische Vorrichtungen mit hohem Brechungsindex, wobei das Material für die ophthalmische Vorrichtung
(i) zumindest ein hydrophobes Arylacrylmonomer der Formel 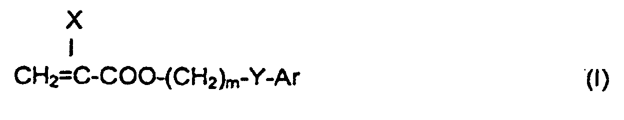 wobei:
X H oder CH₃ ist;
m 0 bis 10 ist;
Y nichts, O, S oder NR ist, worin R H, CH₃, CₙH₂ₙ₊₁ (n = 1 bis 10), iso-OC₃H₇, C₆H₅ oder CH₂C₆H₅ ist;
Ar ein aromatischer Ring ist, der unsubstituiert oder mit CH₃, C₂H₅, n-C₃H₇, iso-C₃H₇, OCH₃, C₆H₁₁, Cl, Br, C₆H₅ oder CH₂C₆H₅ substituiert ist;
(ii) einen Polymerisationsinitiator für die erste Stufe, ausgewählt aus der Gruppe, bestehend aus thermischen Niedrigtemperaturpolymerisationsinitiatoren und Photoinitiatoren; und
(iii) ein Vernetzungsmittel für die zweite Stufe, ausgewählt aus der Gruppe, bestehend aus Dibenzoylperoxid; substituierten Dibenzoylperoxidverbindungen und Dicumylperoxid, umfaßt; und
wobei die erste Stufe des Verfahrens die Polymerisation des Materials für die ophthalmische Vorrichtung durch die Aktivierung des Polymerisationsinitiators für die erste Stufe ohne die Aktivierung des Vernetzungsmittels für die zweite Stufe umfaßt, und die zweite Stufe des Verfahrens die Vernetzung des Materials für die ophthalmische Vorrichtung durch die Aktivierung des Vernetzungsmittels für die zweite Stufe unter Verwendung von Wärme umfaßt, vorausgesetzt, daß das Material für die ophthalmische Vorrichtung keinen Inhaltsstoff enthält, der mehr als eine ungesättigte Bindung aufweist.

2. Verfahren nach Anspruch 1, wobei die hydrophoben Arylacrylmonomere aus der Gruppe ausgewählt sind, bestehend aus 2-Phenoxyethylacrylat; 2-Phenylethylthioacrylat; 2-Phenylethylaminoacrylat; Phenylacrylat; Benzylacrylat; 2-Phenylethyl-acrylat; 3-Phenylpropylacrylat; 3-Phenoxypropylacrylat; 4-Phenylbutylacrylat; 4-Phenoxybutylacrylat; 4-Methylphenylacrylat; 4-Methylbenzylacrylat; 2-2-Methylphenyl-ethylacrylat; 2-3-Methylphenylethylacrylat; 2-4-Methylphenylethylacrylat und deren entsprechenden Methacrylatverbindungen.

3. Verfahren nach Anspruch 1, wobei m 2 bis 4 ist; Y nichts oder O ist und Ar Phenyl ist.

4. Verfahren nach Anspruch 1, wobei das Material für die ophthalmische Vorrichtung zumindest zwei hydrophobe Arylacrylmonomere der Formel (I) umfaßt und worin ferner zumindest eines der hydrophoben Arylacrylmonomere ein Methacrylatmonomer ist und zumindest eines der hydrophoben Arylacrylmonomere ein Acrylatmonomer ist.

5. Verfahren nach Anspruch 4, wobei die Materialien für die ophthalmische Vorrichtung insgesamt zumindest 50 Gew.-% der hydrophoben Arylacrylmonomere der Formel (I) umfassen.

6. Verfahren nach Anspruch 5, wobei die Materialien für die ophthalmische Vorrichtung insgesamt zumindest 70 Gew.-% der hydrophoben Arylacrylmonomere der Formel (I) umfassen.

7. Verfahren nach Anspruch 1, wobei das Material für die ophthalmische Vorrichtung ferner ein oder mehre Monomere, ausgewählt aus der Gruppe, bestehend aus C₁-C₈-Alkylacrylaten, C₁-C₈-Cycloalkylacrylaten, C₁-C₈-N-Alkylacrylamiden, C₁-C₈-Phenoxyalkylacrylaten und deren entsprechenden Methacrylaten, umfaßt.

8. Verfahren nach Anspruch 1, wobei der Polymerisationsinitiator für die erste Stufe aus der Gruppe, bestehend aus 2,2'-Azobis(isobutyronitril); 2,2'-Azobis(2,4-dimethylvaleronitril); UV-Photoinitiatoren und Blaulicht-Photoinitiatoren, ausgewählt ist.

9. Verfahren nach Anspruch 1, wobei der Polymerisationsinitiator für die erste Stufe ein Benzoylphosphinoxid-Initiator ist.

10. Verfahren nach Anspruch 1, wobei die Menge an Polymerisationsinitiator für die erste Stufe etwa 3 Gew.-% oder weniger beträgt.

11. Verfahren nach Anspruch 1, wobei das Vernetzungsmittel für die zweite Stufe aus der Gruppe, bestehend aus Dibenzoylperoxid, 2,4-Dichlordibenzoylperoxid und Dicumylperoxid, ausgewählt ist.

12. Verfahren nach Anspruch 11, wobei das Vernetzungsmittel für die zweite Stufe Dicumylperoxid ist.

13. Verfahren nach Anspruch 1, wobei die Menge an dem Vernetzungsmittel für die zweite Stufe etwa 2 bis 5 Gew.-% beträgt.

14. Verfahren nach Anspruch 1, wobei das Material für ophthalmische Vorrichtungen ferner einen oder mehrere Inhaltsstoffe, ausgewählt aus der Gruppe, bestehend aus UV-absorbierenden Verbindungen und Blaulicht-absorbierenden Verbindungen, umfaßt.

15. Material für ophthalmische Vorrichtungen, erhältlich durch das Verfahren nach Anspruch 1.

## Revendications

1. Procédé à deux stades de préparation d'une matière pour dispositif ophtalmique d'un indice de réfraction élevé, acrylique, dans lequel la matière pour dispositif ophtalmique comprend :
(i) au moins un monomère hydrophobe aryl acrylique de la formule : 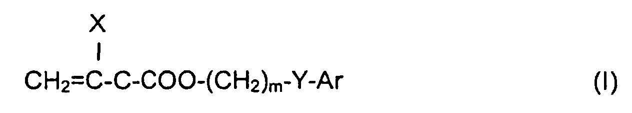 dans laquelle :
X est H ou CH₃;
m est 0-10;
Y est rien, O, S ou NR dans lequel R est H, CH₃, CₙH₂ₙ₊₁ (n=1-10), iso-OC₃H₇, C₆H₅ ou CH₂C₆H₅;
Ar est un noyau aromatique quelconque qui peut être non substitué ou substitué avec CH₃, C₂H₅, n-C₃H₇, iso-C₃H₇, OCH₃, C₆H₁₁, Cl, Br, C₆H₅ ou CH₂C₆H₅;
(ii) un initiateur de polymérisation de premier stade choisi dans le groupe comprenant les initiateurs de polymérisation thermique à basse température et les photo-initiateurs; et
(iii) un agent de réticulation de second stade choisi dans le groupe comprenant le peroxyde de dibenzoyle, les composés de peroxyde de dibenzoyle substitués et le peroxyde de dicumyle; et
dans lequel le premier stade du procédé comprend la polymérisation de la matière pour dispositif ophtalmique par activation de l'initiateur de polymérisation de premier stade sans activer l'agent de réticulation de second stade, et le second stade du procédé comprend la réticulation de la matière pour dispositif ophtalmique par activation de l'agent de réticulation de second stade en utilisant de la chaleur, pour autant que la matière pour dispositif ophtalmique ne contienne pas d'ingrédient comportant plus d'une liaison insaturée.

2. Procédé suivant la revendication 1, dans lequel les monomères hydrophobes aryl acryliques sont choisis dans le groupe comprenant l'acrylate de 2-phénoxyéthyle, le 2-phényléthylthio acrylate, le 2-phényléthylamino acrylate, l'acrylate de phényle, l'acrylate de benzyle, l'acrylate de 2-phényléthyle, l'acrylate de 3-phénylpropyle, l'acrylate de 3-phénoxypropyle, l'acrylate de 4-phénylbutyle, l'acrylate de 4-phénoxybutyle, l'acrylate de 4-méthylphényle, l'acrylate de 4-méthylbenzyle, l'acrylate de 2,2-méthylphényléthyle, l'acrylate de 2,3-méthylphényléthyle, l'acrylate de 2,4-méthylphényléthyle et leurs composés de méthacrylate correspondants.

3. Procédé suivant la revendication 1, dans lequel m est 2-4, Y est rien ou O, et Ar est du phényle.

4. Procédé suivant la revendication 1, dans lequel la matière pour dispositif ophtalmique comprend au moins deux monomères hydrophobes aryl acryliques de formule (I) et de plus dans lequel au moins un des monomères hydrophobes aryl acryliques est un monomère de méthacrylate et au moins un des monomères hydrophobes aryl acryliques est un monomère d'acrylate.

5. Procédé suivant la revendication 4, dans lequel les matières pour dispositif ophtalmique comprennent un total d'au moins 50% (poids/poids) de monomères hydrophobes aryl acryliques de formule (I).

6. Procédé suivant la revendications 5, dans lequel les matières pour dispositif ophtalmique comprennent un total d'au moins 70% (poids/poids) de monomères hydrophobes aryl acryliques de formule (I).

7. Procédé suivant la revendications 1, dans lequel la matière pour dispositif ophtalmique comprend de plus un ou plusieurs monomères choisis dans le groupe comprenant les acrylates d'alkyle en C₁-C₈, les acrylates de cycloalkyle en C₁-C₈, les N-alkylacrylamides en C₁-C₈, les acrylates de phénoxyalkyle en C₁-C₈ et leurs méthacrylates correspondants.

8. Procédé suivant la revendication 1, dans lequel l'initiateur de polymérisation de premier stade est choisi dans le groupe comprenant le 2,2'-azobis(isobutyronitrile), le 2,2'-azobis(2,4-diméthylvaléronitrile), les photo-initiateurs à l'U.V. et les photo-initiateurs à la lumière bleue.

9. Procédé suivant la revendication 1, dans lequel l'initiateur de polymérisation de premier stade est un initiateur d'oxyde de benzoylphosphine.

10. Procédé suivant la revendication 1, dans lequel la quantité d'initiateur de polymérisation de premier stade est d'environ 3% (poids/poids) ou moins.

11. Procédé suivant la revendication 1, dans lequel l'agent de réticulation de second stade est choisi dans le groupe comprenant le peroxyde de dibenzoyle, le peroxyde de 2,4-dichlorodibenzoyle et le peroxyde de dicumyle.

12. Procédé suivant la revendication 11, dans lequel l'agent de réticulation de second stade est le peroxyde de dicumyle.

13. Procédé suivant la revendication 1, dans lequel la quantité d'agent de réticulation de second stade est d'environ 2-5% (poids/poids).

14. Procédé suivant la revendication 1, dans lequel la matière pour dispositif ophtalmique comprend de plus un ou plusieurs ingrédients choisis dans le groupe comprenant les composés absorbant l'U.V. et les composés absorbant la lumière bleue.

15. Matière pour dispositif ophtalmique obtenable par le procédé suivant la revendication 1.
